(19) Europäisches Patentamt / European Patent Office / Office européen des brevets

(11) EP 4 471 880 A1

(12) EUROPEAN PATENT APPLICATION
published in accordance with Art. 153(4) EPC

(43) Date of publication:
04.12.2024 Bulletin 2024/49

(21) Application number: 23746906.9

(22) Date of filing: 23.01.2023

(51) International Patent Classification (IPC):
H01L 33/50 (2010.01)        A61B 5/02 (2006.01)
A61B 5/1455 (2006.01)       F21S 2/00 (2016.01)
G01N 21/01 (2006.01)        G01N 21/3577 (2014.01)
G01N 21/359 (2014.01)       F21Y 115/10 (2016.01)

(52) Cooperative Patent Classification (CPC):
G01N 21/359; A61B 5/02; A61B 5/1455;
F21S 2/00; G01N 21/255; G01N 21/3577;
G01N 21/55; H01L 33/50; F21Y 2115/10;
G01N 2201/062

(86) International application number:
PCT/JP2023/001974

(87) International publication number:
WO 2023/145695 (03.08.2023 Gazette 2023/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.01.2022 JP 2022010604

(71) Applicant: Panasonic Intellectual Property
Management Co., Ltd.
Kadoma-shi, Osaka 571-0057 (JP)

(72) Inventors:
• ABE, Takeshi
  Kadoma-shi, Osaka 571-0057 (JP)
• MOTEGI, Shogo
  Kadoma-shi, Osaka 571-0057 (JP)
• NITTA, Mitsuru
  Kadoma-shi, Osaka 571-0057 (JP)
• SHIGITANI, Ryosuke
  Kadoma-shi, Osaka 571-0057 (JP)

(74) Representative: Müller-Boré & Partner
Patentanwälte PartG mbB
Friedenheimer Brücke 21
80639 München (DE)

(54) **LIGHT-EMITTING DEVICE AND SENSING SYSTEM**

(57) A light-emitting device (2) that emits output light (L), is a light-emitting device that includes: a light-emitting element (20) that emits a primary light (L1); and a wavelength converter that converts at least a portion of the primary light (L1) into a secondary light (L2). The output light (L) includes at least a portion of the secondary light (L2). The output light (L) has a light intensity greater than or equal to a predetermined value throughout an entire wavelength range of from 750 nm to 900 nm, inclusive. A ratio of a light intensity of the output light (L) at 900 nm to a light intensity of the output light (L) at 750 nm is greater than or equal to 0.2 and less than 3.0. The output light (L) has a spectral luminosity of at least 0.1 lm/W and at most 10 lm/W.

FIG. 3

# Description

[Technical Field]

The present invention relates to a light-emitting device and a sensing system.

[Background Art]

Patent Literature (PTL) 1 discloses a beam-emitting optoelectronic element provided in a spectrometer for use in the analysis of organic material or the like. The optoelectronic element includes a conversion substance that converts a primary beam, emitted by a semiconductor chip, to a secondary beam of a wavelength between 700 nm to 2,000 nm.

[Citation List]

[Patent Literature]

[PTL 1] WO 2016/174236

[Summary of Invention]

[Technical Problem]

An object of the present invention is to provide a light-emitting device and a sensing system capable of assisting in highly accurate measurement of oxygen saturation and the like.

[Solution to Problem]

A light-emitting device according to one aspect of the present invention is a light-emitting device that emits output light and the light-emitting device includes: a light-emitting element that emits a primary light; and a wavelength converter that converts at least a portion of the primary light into a secondary light. The output light includes at least a portion of the secondary light. The output light has a light intensity greater than or equal to a predetermined value throughout an entire wavelength range of from 750 nm to 900 nm, inclusive. A ratio of a light intensity of the output light at 900 nm to a light intensity of the output light at 750 nm is greater than or equal to 0.2 and less than 3.0. The output light has a spectral luminosity of at least 0.1 lm/W and at most 10 lm/W.

A sensing system according to one aspect of the present invention includes the above-mentioned light-emitting device according to the one aspect of the present invention, and a detection device that detects reflected light of the above-mentioned output light.

[Advantageous Effects of Invention]

The present invention can assist in highly accurate measurement of oxygen saturation and the like.

[Brief Description of Drawings]

> [FIG. 1]

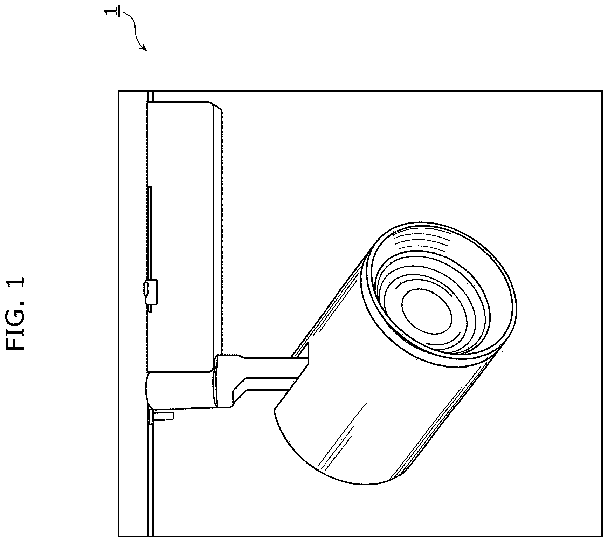

> FIG. 1 is a perspective view illustrating an example of an appearance of a light-irradiating device that includes a light-emitting device according to Embodiment 1.
> [FIG. 2]

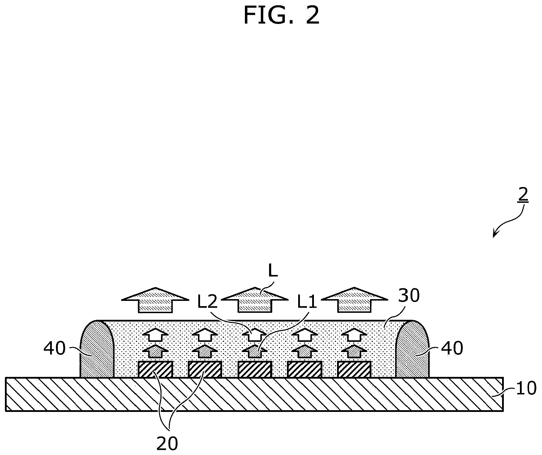

> FIG. 2 is a cross-sectional view of the light-emitting device according to Embodiment 1.
> [FIG. 3]

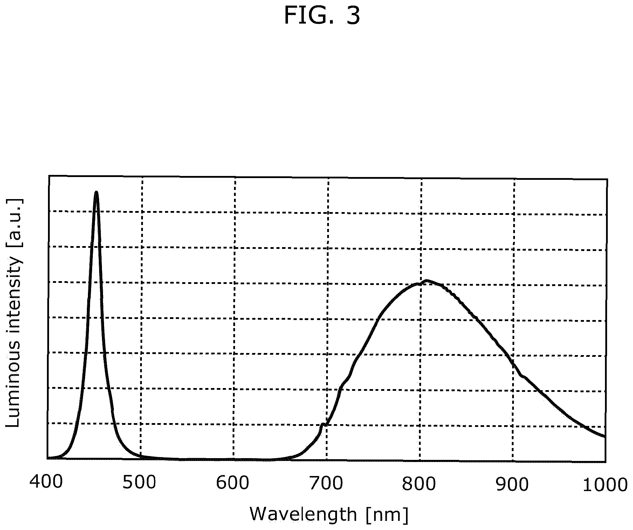

> FIG. 3 is a diagram illustrating an example of a spectrum of output light emitted by the light-emitting device according to Embodiment 1.
> [FIG. 4]

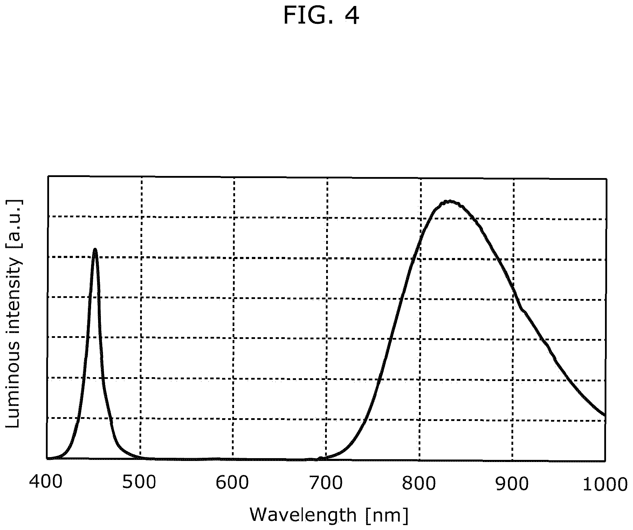

> FIG. 4 is a diagram illustrating another example of a spectrum of output light emitted by the light-emitting device according to Embodiment 1.
> [FIG. 5]

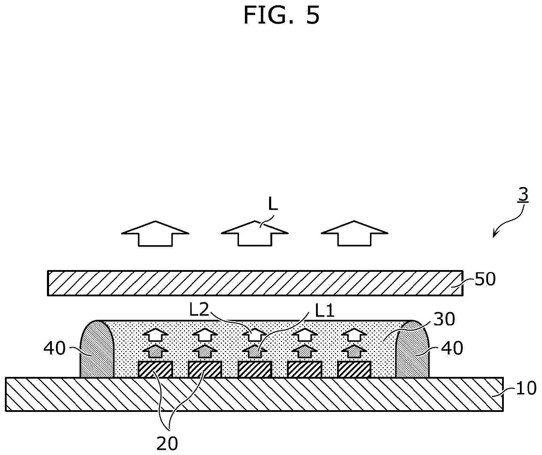

> FIG. 5 is a cross-sectional view of a light-emitting device according to Embodiment 2.
> [FIG. 6]

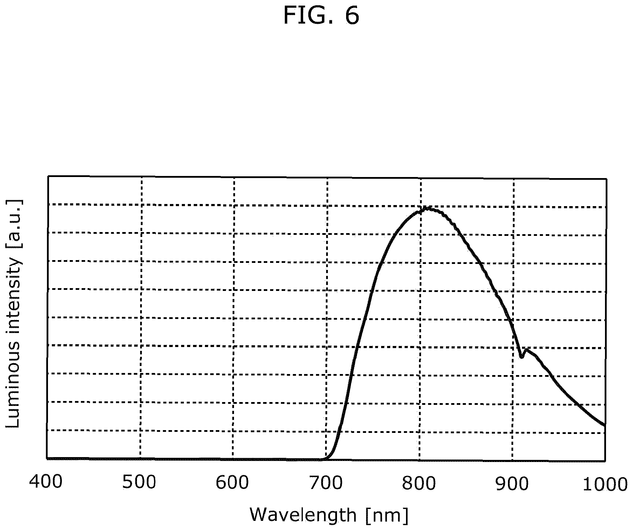

> FIG. 6 is a diagram illustrating an example of a spectrum of output light emitted by the light-emitting device according to Embodiment 2.
> [FIG. 7]

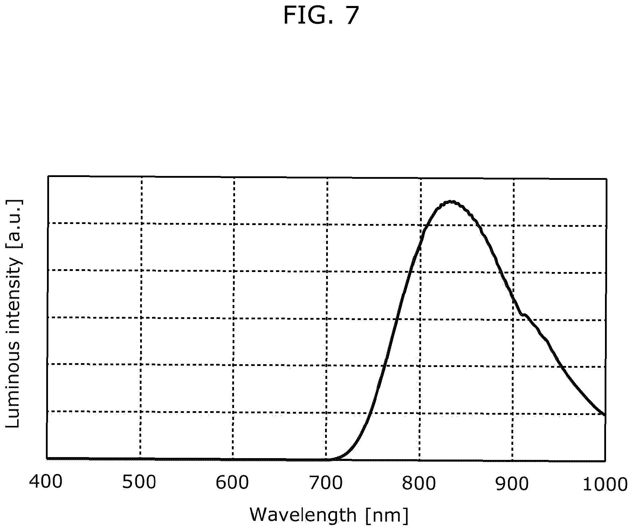

> FIG. 7 is a diagram illustrating another example of a spectrum of output light emitted by the light-emitting device according to Embodiment 2.
> [FIG. 8]

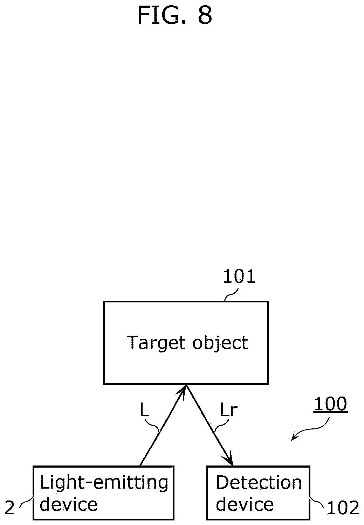

> FIG. 8 is a diagram illustrating a configuration of a sensing system according to Embodiment 3.

[Description of Embodiments]

Hereinafter, light-emitting devices, sensing systems, and the like according to embodiments of the present invention will be described in detail with reference to the drawings. It should be noted that the embodiments described below each illustrate a specific example of the present invention. The numerical values, shapes, materials, elements, the arrangement and connection of the elements, steps, the order of the steps, etc., described in the following embodiments are mere examples, and are therefore not intended to limit the present invention. Accordingly, among elements in the following embodiments, those not appearing in any of the independent claims will be described as optional elements.

It should be noted that the figures are schematic diagrams and are not necessarily precise illustrations. Therefore, for example, the scaling, and so on, depicted in the drawings is not necessarily uniform. Moreover, elements that are substantially the same are given the same reference signs in the respective figures, and redundant descriptions may be omitted or simplified.

Furthermore, in the present specification, terms indicating relationships between elements, terms indicating shapes of elements, and numerical ranges refer not only to their strict meanings, but also include variations that fall within an essentially equivalent range, such as a range of deviation of a few percent.

(Embodiment 1)

[Configuration]

First, a configuration of a light-emitting device according to Embodiment 1 will be described with reference to FIG. 1 and FIG. 2.

FIG. 1 is a perspective view illustrating an example of an appearance of light-irradiating device 1 that includes a light-emitting device according to the present embodiment. FIG. 2 is a cross-sectional view of light-emitting device 2 according to the present embodiment.

Light-irradiating device 1 illustrated in FIG. 1 is, for example, a spotlight, and includes light-emitting device 2 illustrated in FIG. 2. Light-irradiating device 1 includes light-emitting device 2, a housing that houses light-emitting device 2, and an optical system, such as a lens or the like that allows light emitted by light-emitting device 2 to pass through. Light-irradiating device 1 is fixed to a track lighting rail or the like installed on a ceiling. Light-irradiating device 1 receives power supplied by a power supply device, such as a commercial power supply or the like, and emits output light L (see FIG. 2) emitted by light-emitting device 2 using the power supplied. It should be noted that light-irradiating device 1 may be a downlight, ceiling light, pendant light, wall light, or a floor light.

Light-emitting device 2 emits output light L. Output light L includes primary light L1 and secondary light L2. In other words, output light L is composite light that includes primary light L1 and secondary light L2. Primary light L1 is visible light and is blue light, for example. Secondary light L2 is near-infrared light. The specific spectrum of output light L will be described later.

As illustrated in FIG. 2, light-emitting device 2 includes substrate 10, light-emitting elements 20, wavelength converter layer 30 that includes a wavelength converter, and dam material 40.

Substrate 10 is a substrate for mounting light-emitting elements 20. Metal wiring (not illustrated in the drawings) is provided in substrate 10 for supplying power to the plurality of light-emitting elements 20. Substrate 10 is, for example, an insulating substrate, such as a ceramic substrate made of ceramic, a resin substrate made of resin, a glass substrate, or the like. Alternatively, substrate 10 may be a metal-based substrate (metal substrate) that includes a metal plate coated by an insulating layer.

Light-emitting elements 20 emit primary light L1. Light-emitting elements 20 are, for example, light-emitting diode (LED) chips and are mounted on substrate 10. Light-emitting elements 20 are, for example, blue-light LED chips with a central wavelength (peak wavelength of a light-emitting spectrum) in a range of from 430 nm to 495 nm, inclusive. For example, light-emitting elements 20 are blue-light LED chips that emit blue light with a peak wavelength of approximately 440 nm as primary light L1.

The output energy of primary light L1 per each light-emitting element 20 is, for example, 5 mW or more, but may be 10 mW or more, or may be 30 mW or more. The total output energy of primary light L1 output by all light-emitting elements 20 included in light-emitting device 2 is 100 mW or more, for example. The total output energy of primary light L1 may be 1 W or more, 1.2 W or more, 5 W or more, or 10 W or more. As the output energy of primary light L1 increases, the output energy of secondary light L2 also increases.

Wavelength converter layer 30 includes a wavelength converter that converts at least a portion of primary light L1 into secondary light L2. Wavelength converter layer 30 includes, for example, a resin layer, and a wavelength converter is dispersed inside the resin layer. The resin layer is light-transmissive with respect to visible light and near-infrared light. For example, the resin layer may be formed using silicone resin, but this example is non-limiting.

In the present embodiment, the wavelength converter includes one or two or more types of phosphors. At least one type of phosphor among the one or two or more types of phosphors is a near-infrared phosphor that emits near-infrared light. In other words, secondary light L2 includes light in a near-infrared band.

A phosphor with a fluorescence intensity peak that is within a wavelength range of from 780 nm to less than 2,500 nm, for example, and preferably from 800 nm to less than 2,500 nm can be used as a near-infrared phosphor. Accordingly, output light L can include infrared light components that are not visible to the human eye. Furthermore, in this case, a light that exhibits a maximum intensity value that is within a wavelength range of visible light from 380 nm to less than 700 nm (blue light, for example) can be used as primary light L1 that is absorbed by the near-infrared phosphor.

It should be noted that a phosphor with a peak fluorescence intensity less than 780 nm can be used as a near-infrared phosphor, as long as the phosphor used has a certain degree of fluorescence intensity that is within a wavelength range of from at least 780 nm to less than 2,500 nm.

A phosphor that is activated by at least one of rare-earth ions or transition-metal ions and emits fluorescent light that includes near-infrared light components can be used as a near-infrared phosphor. Rare-earth ions include at least one ion selected from a group including $Nd^{3+}$, $Eu^{2+}$, $Ho^{3+}$, $Er^{3+}$, $Tm^{3+}$, and $Yb^{3+}$. Transition-metal ions include at least one ion selected from a group including $Ti^{3+}$, $V^{4+}$, $Cr^{4+}$, $V^{3+}$, $Cr^{3+}$, $V^{2+}$, $Mn^{4+}$, $Fe^{3+}$, $Co^{3+}$, $Co^{2+}$, and $Ni^{2+}$. Furthermore, a phosphor that includes one of the above-mentioned ions as a luminescent center element and includes at least one of an oxide, a sulfide, a nitride, a halide, an oxysulfide, an oxynitride, or an oxyhalide as a host material can be used as a near-infrared phosphor.

In the present embodiment, a phosphor that includes $Cr^{3+}$, for example, can be used as a near-infrared phosphor. Accordingly, a near-infrared phosphor that has a property to absorb visible light, and in particular blue light or red light, and convert such light to near-infrared light components can be easily realized. Furthermore, depending on the type of the host material, the peak wavelength of light absorbed and/or the peak wavelength of fluorescent light can be easily changed, which acts as an advantage when changing the excitation spectrum shape and/or the fluorescent light shape.

Furthermore, a great number of $Cr^{3+}$ activated phosphors that absorb blue light or red light and convert such light to near-infrared fluorescent components are known. Accordingly, not only is the range of choices from which light-emitting elements 20 are selected further widened, but since the peak wavelength of fluorescent light of secondary light L2 becomes easy to change, there is an advantage in controlling the spectral distribution of output light L.

For example, a composite oxide phosphor that includes a garnet crystal structure and is activated by $Cr^{3+}$ can be used as a near-infrared phosphor. Such $Cr^{3+}$ activated garnet phosphors are, for example, at least one of a rare-earth aluminum garnet phosphor or a rare-earth gallium garnet phosphor. Specifically, a $Cr^{3+}$ activated garnet phosphor is, for example, at least one phosphor chosen from a group that includes $Y_3Al_2(AlO_4)_3{:}Cr^{3+}$, $La_3Al_2(AlO_4)_3{:}Cr^{3+}$, $Gd_3Al_2(AlO_4)_3{:}Cr^{3+}$, $Y_3Ga_2(AlO_4)_3{:}Cr^{3+}$, $La_3Ga_2(AlO_4)_3{:}Cr^{3+}$, $Gd_3Ga_2(AlO_4)_3{:}Cr^{3+}$, $Y_3Sc_2(AlO_4)_3{:}Cr^{3+}$, $La_3Sc_2(AlO_4)_3{:}Cr^{3+}$, $Gd_3Sc_2(AlO_4)_3{:}Cr^{3+}$, $Y_3Ga_2(GaO_4)_3{:}Cr^{3+}$, $La_3Ga_2(GaO_4)_3{:}Cr^{3+}$, $(Gd,La)_3Ga_2(GaO_4)_3{:}Cr^{3+}$, $Gd_3Ga_2(GaO_4)_3{:}Cr^{3+}$, $Y_3Sc_2(GaO_4)_3{:}Cr^{3+}$, $La_3Sc_2(GaO_4)_3{:}Cr^{3+}$, $Gd_3Sc_2(GaO_4)_3{:}Cr^{3+}$, and $(Gd,La)_3(Ga,Sc)_2(GaO_4)_3{:}Cr^{3+}$. Furthermore, the $Cr^{3+}$ activated garnet phosphor may be a solid solution that includes these phosphors as end-members.

Additionally, a $Cr^{3+}$ activated phosphor with a crystal structure other than garnet may be used as a near-infrared phosphor. Since $Cr^{3+}$ activated phosphors with a crystal structure other than garnet tend to have a peak fluorescence intensity that is in a wavelength range that starts from 820 nm, a light-emitting device that emits light that can be used to perform highly accurate measurements of oxygen saturation and the like can be provided.

Such near-infrared phosphors are highly durable to high output usage. As the output energy of excitation light (primary light L1) increases, heat generated when wavelength conversion is performed by a near-infrared phosphor becomes more plentiful, thereby increasing the temperature. The above-mentioned near-infrared phosphors are resistant to degradation at high temperatures, and can efficiently convert primary light L1, which has a high output energy, to secondary light L2.

The output energy of secondary light L2, which is given off by wavelength converter layer 30, is 10 mW or more, for example. The output energy of secondary light L2 may be 100 mW or more, 1 W or more, 5 W or more, or 10 W or more. As the output energy of secondary light L2 increases, accuracy of measuring vital signs, such as oxygen saturation and the like can be increased.

Note that although, in the present embodiment, an example is given where wavelength converter layer 30 is disposed in such a manner that a plurality of light-emitting elements 20 are collectively sealed within, this example is non-limiting. A plurality of wavelength converter layers 30 may be disposed separately for each single light-emitting element 20 or for a plurality of light-emitting elements 20. When wavelength converter layers 30 are provided at the locations of each light-emitting element 20, the output energy of secondary light L2 given off by wavelength converter layer 30 for each single light-emitting element 20 is, for example, 5 mW or more. Alternatively, the output energy of secondary light L2 given off by each single wavelength converter layer 30 may be 10 mW or more, or 30 mW or more.

Dam material 40 is a component provided on substrate 10 for enclosing wavelength converter layer 30. In the present embodiment, dam material 40 is provided in an annular shape so as to surround the periphery of wavelength converter layer 30. It should be noted that dam material 40 need not be provided.

Dam material 40 is, for example, formed using thermosetting resin or thermoplastic resin that have insulating properties, or the like. Specifically, silicone resin, phenol resin, epoxy resin, or the like is used as dam material 40.

Light-emitting device 2 as configured above emits output light L that includes near-infrared light. Output light L that includes near-infrared light can be used for measuring vital signs (non-contact sensing), such as oxygen saturation in a person's blood, pulse waves, and the like. For example, light absorption of hemoglobin in blood is used to measure oxygen saturation. Specifically, absorbance curves, which represent the respective absorbances for each wavelength in hemoglobin bonded to oxygen ($HbO_2$) and hemoglobin separated from oxygen (Hb), are different. For example, at a wavelength of 750 nm and in the vicinity of a wavelength of 750 nm, the absorbance of Hb is higher than the absorbance of $HbO_2$. Furthermore, at a wavelength of 900 nm and in the vicinity of 900 nm, the absorbance of $HbO_2$ is higher than the absorbance of Hb.

Accordingly, by emitting output light L that includes these two wavelengths of light, receiving reflected light reflected by a blood vessel, and calculating a ratio of the intensity of the light received, the ratio between Hb and $HbO_2$, or in other words, oxygen saturation can be calculated. Furthermore, in the same manner, by detecting changes over time in arterial blood, pulse waves can also be measured. By emitting a light with a sufficient intensity, the influence of noise and the like can be suppressed, and accuracy of measurement of vital signs, such as oxygen saturation, pulse waves, and the like can be increased.

[Output Light Spectrum]

Next, spectrums of output light L will be described with reference to FIG. 3 and FIG. 4.

FIG. 3 and FIG. 4 are each a diagram illustrating an example of a spectrum of output light L emitted by light-emitting device 2 according to the present embodiment. In each diagram the horizontal axis represents wavelength (unit: nm) and the vertical axis represents luminous intensity.

Both FIG. 3 and FIG. 4 each represent a spectrum of output light L emitted by light-emitting device 2 that includes, as light-emitting element 20, a blue-light LED chip of the same type. The blue-light LED chip emits blue light, which has a peak wavelength of approximately 450 nm and a half bandwidth of approximately 15 nm, as primary light L1. In the present embodiment since output light L includes primary light L1, there is a light intensity peak in the blue light wavelength band as illustrated in FIG. 3 and FIG. 4.

The light-emitting device 2 that emits the output light L illustrated in FIG. 3 and the light-emitting device 2 that emits the output light L illustrated in FIG. 4 include different types of wavelength converters. Specifically, light-emitting device 2 that emits the output light L illustrated in FIG. 3, includes $(Ga,Sc)_2O_3:Cr^{3+}$ and $Gd_3Ga_2(GaO_4)_3:Cr^{3+}$, as wavelength converters, at a volume ratio of 4:6. The light-emitting device 2 that emits the output light L illustrated in FIG. 4, includes only $(Ga,Sc)_2O_3:Cr^{3+}$ as a wavelength converter. It should be noted that each of the wavelength converters are diffused within silicone resin. As illustrated in FIG. 3 and FIG. 4, each of the peaks that correspond to secondary light L2 emitted by the wavelength converters can be found in the near-infrared wavelength band.

In the present embodiment, output light L (secondary light L2) has a light intensity greater than or equal to a predetermined value throughout an entire wavelength range that spans from 750 nm to 900 nm, inclusive. In other words, output light L includes a broad range of near-infrared components.

The predetermined value is, for example, 0.05 times peak intensity or more. The predetermined value may be 0.1 times or more of peak intensity, 0.2 times or more of peak intensity, 0.3 times or more of peak intensity, 0.4 times or more of peak intensity, or 0.5 times or more of peak intensity. It should be noted that the peak intensity is the maximum peak intensity found to be included in a wavelength range of from 750 nm to 900 nm, inclusive. As the predetermined value increases, the intensity of the near-infrared light components included in output light L increases, and thus, the accuracy of measurement of oxygen saturation and the like can be enhanced.

As an optical receiver (photodetector), for example, is used for measurement, even if the characteristics of the optical receiver change, degradation of the accuracy of measurement can be inhibited. In other words, since stringent measurement conditions are not demanded of the characteristics of the optical receiver, light-emitting device 2 can be applied to sensing systems that have a high level of general-purpose versatility.

In the present embodiment, a ratio of the light intensity of output light L at 900 nm to the light intensity of output light L at 750 nm is from 0.2 to less than 3.0. The ratio of the light intensity of output light L at 900 nm to the light intensity of output light L at 750 nm may be 1.0. Accordingly, since a difference in intensity between each of the wavelengths does not need to be taken into account, signal processing can be made simpler. Alternatively, the ratio of the light intensity of output light L at 900 nm to the light intensity of output light L at 750 nm may be from 0.2 to less than 1.0, or may be greater than 1.0 and less than 3.0. Depending on the target object being measured, a desired spectrum can be achieved by adjusting the type of wavelength converter and the mixture ratio of the wavelength converter.

For example, in the example illustrated in FIG. 3, the peak wavelength of secondary light L2 is approximately 810 nm. Assuming the light intensity at the peak wavelength (i.e., peak intensity) is 1, the light intensity at 750 nm is approximately 0.74, and the light intensity at 900 nm is approximately 0.54. In other words, the light intensity at 900 nm is lower than the light intensity at 750 nm. Specifically, the ratio of the light intensity of output light L at 900 nm to the light intensity of output light L at 750 nm is approximately 0.73.

Furthermore, in the example illustrated in FIG. 4, the peak wavelength of secondary light L2 is approximately 830 nm. Assuming the light intensity at the peak wavelength is 1, the light intensity at 750 nm is approximately 0.22, and the light intensity at 900 nm is approximately 0.66. In other words, the light intensity at 900 nm is higher than the light intensity at 750 nm. Specifically, the ratio of the light intensity of output light L at 900 nm to the light intensity of output light L at 750 nm is approximately 3.0.

In the present embodiment, spectral luminosity of output light L is from 0.1 lm/W to 10 lm/W, inclusive. Spectral luminosity is a function in which wavelength $\lambda$ is a variable, and is represented as $K(\lambda)$. Spectral luminosity $K(\lambda)$ is represented in Equation 1 below.

$$(1)\ K(\lambda) = KmV(\lambda)$$

$V(\lambda)$ is relative luminosity, which specifically refers to the luminosity of photopic vision. Here, $Km = 638$ lm/W.

Spectral luminosity $K(\lambda)$ is equivalent to a coefficient for converting physical light intensity to psychophysically perceived brightness. Where spectral density of light intensity is e(λ), brightness as perceived by the human eye f(λ) can be represented in Equation 2 below by using spectral luminosity K(λ).

$$(2)\ f(\lambda) = K(\lambda)e(\lambda)$$

When spectral luminosity K(λ) is too large, the influence of spectral luminosity K(λ) on spatial design increases. That is to say, the influence of visible light components of light emitted by light-emitting device 2 increases, and thus, there is a risk that a person may be given a different impression of an existing space that is illuminated. On the other hand, when spectral luminosity K(λ) is too small, since the visible light components of light emitted by light-emitting device 2 will become negligible, it will not be possible to visually check whether or not light-emitting device 2 is emitting light. In light-emitting device 2 according to the present embodiment, since spectral luminosity K(λ) is in a range of from 0.1 lm/W to 10 lm/W, inclusive, it is possible to visually check whether or not light is being emitted, and the influence on spatial design can be suppressed.

It should be noted that since there are cases where, depending on the lighting environment of the space, it is not possible to visually check whether or not light is being emitted by light-emitting device 2 even when spectral luminosity K(λ) is in a range of from 0.1 lm/W to 10 lm/W, inclusive, spectral luminosity K(λ) may be in a range of from 0.1 lm/W to 20 lm/W, inclusive, or may be in a range of from 0.1 lm/W to 30 lm/W, inclusive.

[Advantageous Effects, etc.]

As described above, light-emitting device 2 according to the present embodiment is a light-emitting device that emits output light L, and includes light-emitting element 20 that emits primary light L1 and a wavelength converter that converts at least a portion of primary light L1 into secondary light L2. Output light L includes at least a portion of secondary light L2. Output light L has a light intensity greater than or equal to a predetermined value throughout an entire wavelength range of from 750 nm to 900 nm, inclusive. The ratio of a light intensity of output light L at 900 nm to a light intensity of output light L at 750 nm is greater than or equal to 0.2 and less than 3.0. Output light L has a spectral luminosity of at least 0.1 lm/W and at most 10 lm/W.

Accordingly, highly accurate measurements of oxygen saturation and the like can be facilitated. Specifically, since output light L has a high light intensity throughout an entire wavelength range that spans from 750 nm to 900 nm, even if the characteristics of the optical receiver changes, degradation of the accuracy of measurement can be inhibited. In other words, since stringent measurement conditions are not demanded of the characteristics of the optical receiver, light-emitting device 2 can be applied to sensing systems that have a high level of general-purpose versatility. Furthermore, since spectral luminosity K(λ) is in a range of from 0.1 lm/W to 10 lm/W, inclusive, it is possible to visually check whether or not light is being emitted, and the influence on spatial design can be suppressed.

Additionally, the wavelength converter may, for example, include two or more types of phosphors.

Accordingly, by using multiple types of phosphors, the spectrum of secondary light L2 can be easily adjusted. For example, an optimal spectrum of secondary light L2 can be obtained in accordance with the measurement target object and/or measurement environment.

Furthermore, at least one type of phosphor among the two or more types of phosphors is a near-infrared phosphor that includes $Cr^{3+}$, for example.

Accordingly, a wavelength converter that is resistant to degradation at a high temperature can be realized. For this reason, the output energy of primary light L1 that is excitation light can be used, and thus, the desired output light L can be emitted in a highly efficient manner. Furthermore, since the output energy of primary light L1 emitted by a single light-emitting element 20 can be increased, the number of light-emitting elements 20 can be reduced. Accordingly, light-emitting device 2 can be miniaturized.

Furthermore, secondary light L2 may, for example, have output energy that is greater than or equal to 100 mW.

Accordingly, since the intensity of near-infrared light can be increased, the accuracy of measurement of oxygen saturation and the like can be increased.

(Embodiment 2)

Next, Embodiment 2 will be described.

A light-emitting device according to Embodiment 2 differs with the light-emitting device according to Embodiment 1 in that the light-emitting device according to Embodiment 2 includes a filter. Hereinafter, the description will focus on the points of difference with Embodiment 1, and overlapping description will be omitted or simplified.

FIG. 5 is a cross-sectional view of light-emitting device 3 according to the present embodiment. In addition to the configuration of light-emitting device 2 illustrated in FIG. 2, light-emitting device 3 illustrated in FIG. 5 includes filter 50.

Filter 50 absorbs or reflects at least a portion of at least one of primary light L1 or secondary light L2. In the present embodiment, filter 50 is a filter that blocks visible light and absorbs or reflects nearly all of primary light L1 and the visible light components present in secondary light L2. Accordingly, output light L that passes through via filter 50 does not include primary light L1 and has a spectrum that is nearly the same as secondary light L2.

Filter 50 includes light-absorbing dyes diffused inside of a base material that includes resin, glass, or the like, for example. Alternatively, filter 50 includes a reflective film, which is provided on the surface of the base material, that reflects visible light components. The reflective film may, for example, be a multi-layer dielectric film. As long as the desired wavelength components can be prevented from passing through, filter 50 is not limited to any particular configuration.

In the example illustrated in FIG. 5, although filter 50 is disposed spaced apart from wavelength converter layer 30, wavelength converter layer 30 and filter 50 may be in contact with each other.

Next, the spectrum of output light L emitted by light-emitting device 3 will be described with reference to FIG. 6 and FIG. 7.

FIG. 6 and FIG. 7 are each a diagram that illustrates an example of a spectrum of output light L that is emitted by light-emitting device 3 according to the present embodiment. In each diagram the horizontal axis represents wavelength (unit: nm) and the vertical axis represents luminous intensity.

The spectrum of the output light L illustrated in FIG. 6 is the spectrum of light of the output light L illustrated in FIG. 3 after output light L has passed through filter 50. That is to say, in the same manner as in FIG. 3, light-emitting device 3 that emits the output light L illustrated in FIG. 6 includes, as a wavelength converter, $(Ga,Sc)_2O_3:Cr^{3+}$ and $Gd_3Ga_2(GaO_4)_3:Cr^{3+}$ at a volume ratio of 4:6.

In the same manner, the spectrum of the output light L illustrated in FIG. 7 is the spectrum of light of the output light L illustrated in FIG. 4 after the light has passed through filter 50. That is to say, in the same manner as in FIG. 4, light-emitting device 3 that emits the output light L illustrated in FIG. 7 only includes $(Ga,Sc)_2O_3:Cr^{3+}$ as a wavelength converter.

As illustrated in FIG. 6 and FIG. 7 output light L that passes through filter 50 includes virtually no light that is in a wavelength range of from 400 nm to 700 nm, inclusive. Output light L includes trace amounts of visible light components that are in a range of from 700 nm to 750 nm, inclusive. By including trace amounts of visible light components (red light in the example given here), whether or not light-emitting device 3 is emitting light can be more readily checked by visual means.

As described above, light-emitting device 3 according to the present embodiment further includes filter 50 that absorbs or reflects at least a portion of at least one of primary light L1 or secondary light L2.

Accordingly, by sufficiently minimizing the visible light components included in output light L, the influence on spatial design can be suppressed. Even in a case where light-emitting device 3 is installed in a typical environment, such as a living space or the like, rather than a specialized environment, such as a laboratory or the like, light-emitting device 3 can be installed in a manner in which the existing lighting environment is left unaltered. Accordingly, it becomes possible to perform measurement of vital signs, such as a person's oxygen saturation, pulse waves, and the like, in living spaces on a day-to-day basis. Since measurement of vital signs by using near-infrared light can be performed by non-contact means, such measurement can be effortlessly performed while not posing a burden on the person performing measurement and not interfering in the person's daily life.

It should be noted that filter 50 may also absorb or reflect only primary light L1. In this case, output light L would include visible light components included in secondary light L2 (e.g., red light of a wavelength of about 700 nm). That is to say, when light-emitting device 3 is emitting light, not only is light-emitting device 3 capable of emitting near-infrared light, but light-emitting device 3 is also capable of emitting red light that can be visually recognized by people. Accordingly, whether or not light-emitting device 3 is emitting light can be more readily checked by visual means.

(Embodiment 3)

Next, Embodiment 3 will be described.

The sensing system according to embodiment 3 includes the light-emitting device according to Embodiment 1 and the light-emitting device according to Embodiment 2. Hereinafter, the description will focus on the points of difference with Embodiment 1 and Embodiment 2, and overlapping description will be omitted or simplified.

FIG. 8 illustrates a configuration of sensing system 100 according to the present embodiment.

Sensing system 100 illustrated in FIG. 8 measures characteristic values of target object 101. Target object 101 is a living being, such as a person or the like, for example. Sensing system 100 measures a person's vital signs, such as oxygen saturation, pulse waves, and the like.

As illustrated in FIG. 8, sensing system 100 includes light-emitting device 2 and detection device 102. It should be noted that sensing system 100 may include light-emitting device 3 according to Embodiment 2 instead of light-emitting device 2.

Detection device 102 detects reflected light Lr of output light L emitted by light-emitting device 2. Reflected light Lr is light that is transmitted when target object 101 reflects output light L.

As described earlier, light of multiple wavelengths that is included in the near-infrared band is used when measuring oxygen saturation, pulse waves, or the like. Accordingly, detection device 102 includes, for example, a photodetector capable of receiving light of multiple wavelengths. The photodetector outputs an electrical signal that corresponds to the intensity of reflected light Lr received.

A quantum photodetector that detects the electrical charge generated when light becomes incident on a p-n junction of a semiconductor can, for example, be used as the photodetector. Specifically, the photodetector is a photodiode, a phototransistor, a photo-integrated circuit (IC), a charge coupled device (CCD) image sensor, a complementary metal-oxide-semiconductor (CMOS) image sensor, or the like. Furthermore, either a thermal photodetector, which detects changes in electrical characteristics resulting from increases in temperature due to heat generated when light is received, a photosensitive infrared film, or the like can be used as a photodetector. A thermopile, which utilizes a thermoelectric effect, a pyroelectric element, which utilizes a pyroelectric effect, or the like can be used as a thermal photodetector.

Detection device 102 includes a signal processing circuit (not illustrated in the drawings) that processes electrical signals output by the photodetector. The signal processing circuit calculates the ratio between Hb and $HbO_2$, i.e., oxygen saturation by calculating the ratio between the intensities of the respective reflected lights Lr of two different wavelengths that are received by the photodetector, for example. Alternatively, the signal processing circuit may calculate a pulse wave.

As described above, sensing system 100 according to the present embodiment includes light-emitting device 2 and detection device 102 that detects reflected light Lr of output light L.

Accordingly, oxygen saturation, pulse waves, or the like can be measured with a high degree of accuracy.

Furthermore, in sensing system 100, since the influence of sensing system 100 on spatial design is small, light-emitting device 2 can be disposed in a living space. Accordingly, a person can effortlessly measure oxygen saturation and the like in the person's daily life without the need for the person to consciously perform such measurement. In this manner, a sensing system 100 that is highly convenient to the user can be realized.

(Other Embodiments)

Although the light-emitting devices, the sensing systems, and the like according to the present invention have been described based on the above embodiments and the like, the present invention is not limited to the above embodiments.

For example, multiple types of different phosphors may be diffused, combined, and disposed in a single wavelength converter layer 30, or separate layers may be provided for each phosphor. For example, wavelength converter layer 30 may have a multi-layered structure of multiple resin layers, and different types of phosphors may be diffused and disposed in each resin layer. Primary light L1 emitted from the light-emitting elements 20 side sequentially passes through multiple types of phosphors so as to be converted to secondary light L2, and thus conversion efficiency can be enhanced. For example, if secondary light L2 that passes through a first type of phosphor is absorbed by a second type of phosphor, then the first type of phosphor is disposed behind the second type of phosphor in the order in which primary light L1 passes through. Accordingly, secondary light L2 that passes through the first type of phosphor can be allowed to exit without being absorbed by the second type of phosphor.

Furthermore, the light-emitting device may, for example, include a sintered substance that is a wavelength converter instead of a wavelength converter that has been diffused within resin. Specifically, the light-emitting device may include a sintered substance (ceramic phosphor) with particles of a phosphor.

Furthermore, for example, although the above embodiments each describe a chip on board (COB) type of light-emitting device in which an LED chip is directly mounted on a substrate, these examples are non-limiting. For example, the light-emitting device may be a surface-mount device (SMD) type of light-emitting element.

An SMD type of light-emitting element may include an LED chip, a package that has a recessed portion that houses the LED chip, a resin layer that fills the recessed portion, and a wavelength converter diffused in the resin layer. In other words, a wavelength converter may be provided as a portion of an SMD type of light-emitting element.

Furthermore, the light-emitting element need not be a blue-light LED. For example, the light-emitting element may be a violet-light LED, an ultraviolet-light LED, or a red-light LED. In other words, primary light L1 need not be blue light, and may be violet light, ultraviolet light, or red light.

Furthermore, the light-emitting element need not be an LED. For example, the light-emitting element may be a laser element or an organic electroluminescent (EL) element.

Furthermore, for example, the light-emitting device may be used to measure items other than vital signs, such as oxygen saturation, pulse waves, or the like. For example, the light-emitting device may be used as a gas sensor or distance sensor that utilizes infrared light, a surveillance camera, or the like.

Forms obtained through various modifications to each of the foregoing embodiments that may be conceived by those skilled in the art, as well as forms realized by combining elements and functions in each of the foregoing embodiments without departing from the essence of the present invention are included within the scope of the present invention.

[Reference Signs List]

2, 3    light-emitting device
20    light-emitting element
30    wavelength converter layer
50    filter
100    sensing system

102   detection device
L      output light
L1     primary light
L2     secondary light
Lr     reflected light

**Claims**

1. A light-emitting device that emits output light, the light-emitting device comprising:

   a light-emitting element that emits a primary light; and
   a wavelength converter that converts at least a portion of the primary light into a secondary light, wherein
   the output light includes at least a portion of the secondary light,
   the output light has a light intensity greater than or equal to a predetermined value throughout an entire wavelength range of from 750 nm to 900 nm, inclusive,
   a ratio of a light intensity of the output light at 900 nm to a light intensity of the output light at 750 nm is greater than or equal to 0.2 and less than 3.0, and
   the output light has a spectral luminosity of at least 0.1 lm/W and at most 10 lm/W.

2. The light-emitting device according to claim 1, further comprising:
   a filter that absorbs or reflects at least a portion of at least one of the primary light or the secondary light.

3. The light-emitting device according to claim 1 or 2, wherein
   the wavelength converter includes two or more types of phosphors.

4. The light-emitting device according to claim 3, wherein
   at least one type of phosphor among the two or more types of phosphors is a near-infrared phosphor that includes $Cr^{3+}$.

5. The light-emitting device according to any one of claims 1 to 4, wherein
   the secondary light has output energy that is greater than or equal to 100 mW.

6. A sensing system comprising:

   the light-emitting device according to any one of claims 1 to 5; and
   a detection device that detects reflected light of the output light.

FIG. 1

EP 4 471 880 A1

FIG. 2

FIG. 3

FIG. 4

EP 4 471 880 A1

# FIG. 5

FIG. 6

FIG. 7

# FIG. 8

## INTERNATIONAL SEARCH REPORT

| International application No. |
|---|
| **PCT/JP2023/001974** |

### A. CLASSIFICATION OF SUBJECT MATTER

*H01L 33/50*(2010.01)i; *A61B 5/02*(2006.01)i; *A61B 5/1455*(2006.01)i; *F21S 2/00*(2016.01)i; *G01N 21/01*(2006.01)i;
*G01N 21/3577*(2014.01)i; *G01N 21/359*(2014.01)i; *F21Y 115/10*(2016.01)n
FI: H01L33/50; A61B5/02 310B; A61B5/1455; F21S2/00 311; G01N21/01 D; G01N21/3577; G01N21/359; F21Y115:10

According to International Patent Classification (IPC) or to both national classification and IPC

### B. FIELDS SEARCHED

Minimum documentation searched (classification system followed by classification symbols)

H01L33/00-33/64; A61B5/00-5/398

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2017/188121 A1 (ASAHI KASEI KK) 02 November 2017 (2017-11-02) paragraphs [0039]-[0054], fig. 9-12 | 1-6 |
| Y | US 2019/0194539 A1 (EVERLIGHT ELECTRONICS CO., LTD.) 27 June 2019 (2019-06-27) paragraphs [0001]-[0007], [0127]-[0133], fig. 12-16 | 1-6 |
| Y | JP 2014-130688 A (STANLEY ELECTRIC CO., LTD.) 10 July 2014 (2014-07-10) paragraphs [0033], [0034], fig. 4 | 1-6 |
| A | JP 2019-10144 A (SEIKO EPSON CORP.) 24 January 2019 (2019-01-24) | 1-6 |
| A | US 2012/0071739 A1 (NELLCOR PURITAN BENNETT LLC) 22 March 2012 (2012-03-22) | 1-6 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 March 2023** | **04 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/001974**

| Patent document cited in search report | | | Publication date (day/month/year) | Patent family member(s) | | | Publication date (day/month/year) |
|---|---|---|---|---|---|---|---|
| WO | 2017/188121 | A1 | 02 November 2017 | US paragraphs [0065]-[0080], fig. 10-13 | 2019/0069780 | A1 | |
| | | | | EP | 3449825 | A1 | |
| US | 2019/0194539 | A1 | 27 June 2019 | EP | 3502208 | A1 | |
| | | | | CN | 109943332 | A | |
| JP | 2014-130688 | A | 10 July 2014 | (Family: none) | | | |
| JP | 2019-10144 | A | 24 January 2019 | (Family: none) | | | |
| US | 2012/0071739 | A1 | 22 March 2012 | (Family: none) | | | |

Form PCT/ISA/210 (patent family annex) (January 2015)

**REFERENCES CITED IN THE DESCRIPTION**

**Patent documents cited in the description**

- WO 2016174236 A